Europäisches Patentamt

European Patent Office

Office européen des brevets

(11) Veröffentlichungsnummer: **0 137 345 B1**

(12) **EUROPÄISCHE PATENTSCHRIFT**

(45) Veröffentlichungstag der Patentschrift: 05.12.90

(21) Anmeldenummer: 84110973.9

(22) Anmeldetag: 14.09.84

(51) Int. Cl.⁵: **C 07 K 15/14,** C 07 K 3/02, G 01 N 33/531

(54) **Membranassoziierte Proteine (MP2), Verfahren zu ihrer Gewinnung sowie ihre Verwendung.**

(30) Priorität: 23.09.83 DE 3334405

(43) Veröffentlichungstag der Anmeldung:
17.04.85 Patentblatt 85/16

(45) Bekanntmachung des Hinweises auf die
Patenterteilung:
05.12.90 Patentblatt 90/49

(84) Benannte Vertragsstaaten:
AT BE CH DE FR GB IT LI LU NL SE

(56) Entgegenhaltungen:
DE-A-2 445 677
FR-A-2 348 222

CHEMICAL ABSTRACTS, Band 83, Heft 4, 1975,
Seite 12, Zusammenfassung Nr. 23765f,
Columbus, Ohio, US; H. HAUPT et al.: "Isolation
and characterization of 19S-alpha1-glycoprotein
from human erythrocytes and its identification
as placenta protein PP6", & BLUT 1975, 30(4),
277-86

(73) Patentinhaber: **BEHRINGWERKE
Aktiengesellschaft
Postfach 1140
D-3550 Marburg 1 (DE)**

(72) Erfinder: **Bohn, Hans, Dr.
Oberer Eichweg 26
D-3550 Marburg 1 (DE)**

(74) Vertreter: **Klein, Otto, Dr. et al
Hoechst AG Zentrale Patentabteilung Postfach
80 03 20
D-6230 Frankfurt am Main 80 (DE)**

(56) Entgegenhaltungen:
CHEMICAL ABSTRACTS, Band 83, Heft 11, 15.
September 1975, Seite 207, Zusammenfassung
Nr. 92637c, Columbus, Ohio, US; H. BOHN et al.:
"Isolation and characterization of a high
molecular weight placental protein (PP6)", &
ARCH. GYNAEKOL. 1975, 218(2), 131-42

**Beschreibung**

Die Erfindung betrifft eine Gruppe von membranassoziierten Gewebeproteinen aus der menschlichen Plazenta. Sie betrifft weiterhin ein Verfahren zur Gewinnung dieser Proteine. Die Proteine können zur Gewinnung von Antiseren und Antikörpern verwendet werden, die zum Nachweis und zur Bestimmung der Proteine dienen können.

Zwei membranassoziierte, als $MP_1$ und $PP_4$ bezeichnete Proteine sind aus den deutschen Patentanmeldungen 33 14 293 und 33 15 000 bekannt.

Von diesen sind die hier beschriebenen Proteine in ihren physikalischen, chemischen und immunchemischen Eigenschaften verschieden. Sie sind auch nicht mit anderen bekannten Strukturproteinen von menschlichem Gewebe, wie Kollagen, Fibrin, Fibronectin, Actin oder Laminin identisch, von denen sie sich ebenfalls in ihren physikalischen, chemischen oder immunchemischen Eigenschaften unterscheiden.

Die in der vorliegenden Patentanmeldung beschriebene Gruppe von komplex zusammengesetzten membranassoziierten Gewebeproteine ($MP_2$) aus der menschlichen Plazenta, weisen hinsichtlich ihrer Sedimentationskoeffizienten und Molekulargewichte beträchtliche Unterschiede auf, sind jedoch immunchemisch identisch oder teilverwandt und sind auch in ihren sonstigen physikalischen Eigenschaften und in ihrer chemischen Zusammensetzung einander sehr ähnlich.

Durch Gelfiltration lassen sich die $MP_2$-Proteine in vier deutlich voneinander abgesetzte Fraktionen (I bis IV) auftrennen. Am Aufbau dieser Proteine sind insgesamt mindestens vier verschiedene Komponenten, die sich in ihren Antigendeterminanten unterscheiden, beteiligt.

Gegenstand der Erfindung ist eine $MP_2$ genannte Gruppe von Proteinen, gekennzeichnet durch

a) eine elektrophoretische Beweglichkeit im Bereich zwischen der von $\alpha_2$—und der von $\beta_1$—Globulinen;

b) einen isoelektrischen Punkt im pH-Bereich von 4,4—5,0;

c) einen Sedimentationskoeffizienten $S^0_{20w}$ im Bereich von 7 bis über 20 S, wobei die Hauptfraktionen (IV, III, II, I) mit etwa 7, 9, 14 bzw. 20 S sedimentieren;

d) Molekulargewichte im Bereich von 200 000 bis 1 000 000, wobei die Hauptfraktionen (IV, III, II, I) Molekulargewichte um 210 000, 400 000, 750 000 und 1 000 000 aufweisen;

e) einen Extinktionskoeffizienten $E^{1\%}_{1\,cm}$ (280 nm) von 12,5±0,2;

f) einen Kohlenhydratanteil von 8,0±3,2% (Mannose 1,4±0,4%, Fucose 0,4±0,2%, Galaktose 1,2±0,4%, N-Acetyl-Glukosamin 2,6±1,8%, N-Acetyl-Neuraminsäure 2,4±1,4%) und

g) eine Aminosäurenzusammensetzung, in der Asparaginsäure, Glutaminsäure, Leucin, Valin, Serin und Glycin zu den am häufigsten vertretenen Aminosäuren gehören.

Tabelle I zeigt beispielhaft das Ergebnis der Aminosäureanalysen für die Fraktionen II und IV.

TABELLE 1

| Aminosäure | Reste pro 100 reste (mol %) | |
| | Fraktion II | Fraktion IV |
| --- | --- | --- |
| Cystein | 2,80 | 3,55 |
| Methionin | 1,80 | 1,55 |
| Tryptophan | 2,60 | 2,95 |
| Asparaginsäure | 10,75 | 10,15 |
| Threonin | 4,85 | 4,55 |
| Serin | 7,50 | 8,15 |
| Glutaminsäure | 9,20 | 9,70 |
| Prolin | 5,45 | 6,25 |
| Glycin | 6,70 | 7,70 |
| Alanin | 6,40 | 6,05 |
| Valin | 7,35 | 7,30 |
| Isoleucin | 4,90 | 4,65 |
| Leucin | 8,95 | 7,90 |
| Tyrosin | 3,35 | 3,25 |
| Phenylalanin | 4,45 | 3,80 |
| Histidin | 2,45 | 2,65 |
| Lysin | 5,50 | 5,60 |
| Arginin | 4,90 | 4,25 |

Zur Erläuterung der kennzeichnenden Merkmale der $MP_2$-Gewebeproteine sei folgendes aufgeführt:

Die elektrophoretische Beweglichkeit wurde mit dem Gerät Microzone R 200 von Beckman Instruments auf Zelluloseazetatfolien (Firma Sartorius) unter Verwendung von Natriumdiäthylbarbiturat-Puffer, pH 8,6, bestimmt.

Der isoelektrische Punkt wurde mit einer Säule (440 ml) der Firma LKB, Stockholm, ermittelt. Das Ampholin®-Gemisch hatte einen pH-Bereich von 4,0 bis 6,0.

Der Sedimentationskoeffizient wurde in einer analytischen Ultrazentrifuge der Firma Beckman (Spinco-Apparatur, Modell E) bei 60.000 UpM in Doppelsektorzellen mit Hilfe der UV-Scanner-Technik bei 280 nm bestimmt. Als Lösungsmittel diente Wasser. Die Proteinkonzentration betrug 2 g/l.

Zur Bestimmung oder Abschätzung der Molekulargewichte wurde das Verhalten bei der Gelfiltration und in der Ultrazentrifuge sowie die elektrophoretische Wanderung im SDS-haltigen Polyacrylamidgel herangezogen. Bei der letzteren Methode wurden Gele mit 7,5 oder 10 g/100 ml Polyacrylamid (PAA) verwendet, die 0,1 g/100 ml Natriumdodecylsulfat (SDS) enthielten. Zur Bestimmung der nativen Molekulargewichte wurden die $MP_2$-Fraktionen I—IV in Wasser oder in 0,01 mol/l Trispuffer gelöst aufgetragen. Als Vergleichssubstanzen dienten Ferritin (MG 450 000), die Untereinheit S (MG 180 000) des fibrinstabilisierenden Faktors, das schwangerschafts-spezifische $\beta_1$-Glykoprotein (MG 90 000) sowie Humanalbumin.

Zur Bestimmung des Extinktionskoeffizienten wurde 1 mg Substanz in destilliertem Wasser zu 1 ml Lösung gelöst.

Die Kohlenhydrate wurden wie folgt bestimmt:

Nach Hydrolyse der glykosidischen Bindungen wurden die freigesetzten Neutralzucker als Boratkomplexe über eine Anionenaustauschersäule getrennt (Y.C. Lee et al., Anal. Biochem. 27 (1969), 567), im Eluat durch Zumischung von Cu(I)-bicinchoninatreagenz (K. Mopper und M. Gindler, Anal. Biochem. 56 (1973), 440) angefärbt und unter Verwendung von Rahmnose als internem Standard quantitativ bestimmt. Die Aminozucker wurden durch ihre Reaktion mit Ninhydrin nachgewiesen und bestimmt. Der Neuraminsäuregehalt ist mit der Methode nach Warren (Methods in Enzymology, Vol. VI (1963), 463—465) ermittelt worden.

Die Aminosäurenanalysen wurden nach S. Moore, D. H. Spackman, W. H. Stein, Anal. Chem. 30 (1958), 1185, unter Verwendung des Flüssigkeitschromatographen Multichrom B der Firma Beckman durchgeführt. Cystin wurde nach Oxydation der Proteine mit Perameisensäure (S. Moore et al., Anal. Chem. 30 (1958), 1185) und nachfolgender Chromatographie (S. Moore, J. Bio. Chem. 238 (1963), 235) als Cysteinsäure bestimmt. Der Tryptophangehalt ist direkt photometrisch nach H. Edelhoch, Biochemistry 6 (1967), 1948, ermittelt worden.

Gegenstand der Erfindung ist weiter ein Verfahren zur Gewinnung der membran-assoziierten Plazentaproteine $MP_2$, dadurch gekennzeichnet, daß man

1. ausgewachsene menschliche Plazenten, wie sie bei der Entbindung anfallen, zerkleinert, mit physiologischer Salzlösung wäscht, bis alle löslichen Bestandteile entfernt sind, und den so gewonnenen Geweberückstand mit einer 0,5—5 g eines nicht-ionischen Detergenzes auf 100 ml enthaltenden Lösung, vorzugsweise mit einer 2 ml/100 ml Polyethylenglycol-p-isooctylphenylether enthaltenden Lösung, extrahiert, den Überstand abtrennt, gegen Wasser oder Pufferlösung dialysiert und einengt;

2. die in der erhaltenen Lösung enthaltenen solubilisierten Gewebeantigen der Plazenta durch eine Immunadsorption weiter anreichert;

3. die nach der Immunadsorption an Antigenen angereicherte Lösung durch eine Gelfiltration auftrennt und gleichzeitig von anderen solubilisierten Proteinen der Plazenta ($MP_1$, $PP_4$), die ein kleineres Molekulargewicht haben, weitgehend abtrennt; und

4. gegebenenfalls die in den $MP_2$-Fraktionen gelegentlich noch als Verunreinigungen vorliegenden Begleitproteine (Spuren von Immunglobulinen und des membranassoziierten Proteins $MP_1$ sowie insbesondere Ferritin, das in der $MP_2$-Fraktion III erscheint) durch inverse Immunadsorption, d.h. durch trägergebundene Antikörper gegen diese Begleitproteine, entfernt und so die $MP_2$-Fraktionen frei von Verunreinigungen gewinnt.

Zum Nachweis der $MP_2$-Proteine, etwa in einer Fraktion aus einer Trennoperation, können neben den angegebenen Parametern auch immunchemische Methoden dienen, da die $MP_2$-Proteine antigene Eigenschaften haben.

Am Aufbau der $MP_2$-Proteine sind insgesamt mindestens 4 Komponenten (A, B, C und D) beteiligt, die sich in ihren Antigendeterminanten unterscheiden. Die Verteilung dieser unterschiedlichen Antigenbezirke in den $MP_2$-Fraktionen I—IV zeigt Tabelle 2. Die Fraktionen I—III enthalten alle vier Antigenbezirke. Die Komponente A scheint dabei am stärksten vertreten zu sein. In Fraktion IV dagegen überwiegt die Komponente B. Die Komponente C fehlt praktisch vollkommen in dieser Fraktion.

TABELLE 2

Verteilung der Antigenbezirke A, B, C und D auf die $MP_2$-Fraktionen I—IV

|     | A   | B   | C   | D   |
| --- | --- | --- | --- | --- |
| I   | ++  | +   | +   | (+) |
| II  | ++  | +   | +   | (+) |
| III | ++  | (+) | +   | (+) |
| IV  | +   | ++  | −   | +   |

++ sehr starck, + stark, (+) schwach und—nicht vertreten

Durch Immunisierung von Kaninchen mit den gereinigten Fraktionen I—IV lassen sich dementsprechend Antiseren gewinnen, die in wechselnder Menge Antikörper gegen die Antigenbezirke A, B, C und D oder aber auch nur gegen einen Teil dieser Komponenten enthalten. Durch geeignete Absorption mit entsprechenden Plazentafraktionen oder $MP_2$-Fraktionen lassen sich diese Antiseren gegen den einen oder anderen Antigenbezirk spezifisch machen. Monoclonale Antikörper, die jeweils nur mit einer von den in diesen Proteinen enthaltenen Antigendeterminante reagieren, werden erhalten, indem man Milzzellen von Mäusen, die mit $MP_2$-Proteinen immunisiert wurden, mit Mausmyelomzellen verschmilzt (fusioniert) und dann die gebildeten Hybridzellen selektiert und weiter vermehrt. Ein solches Verfahren ist beispielsweise von Köhler und Milstein in Nature, Vol. 256 (1975), 495—497, beschrieben worden.

Die $MP_2$-Proteine lassen sich demnach verwenden, um Antiseren gegen die Gesamtheit oder gegen einzelne Fraktionen und Komponenten dieser Proteinkomplexe herzustellen oder aber auch um Antikörper zu gewinnen, die jeweils nur mit einer der in diesen Proteinen enthaltenen Antigendeterminanten reagieren (monoklonale Antikörper).

Die Antiseren und Antikörper lassen sich verwenden, die $MP_2$-Proteine oder damit antigenverwandte Proteine in Geweben zu lokalisieren, in Körperflüssigkeiten nachzuweisen und zu bestimmen. Weiterhin können diese Antiseren und Antikörper dazu dienen, Immunadsorbentien herzustellen, mit deren Hilfe sich immunchemisch mit MP$_2$ identische oder mit MP$_2$ teilverwandte Proteine aus Gewebeextrakten oder Körperflüssigkeiten isolieren lassen.

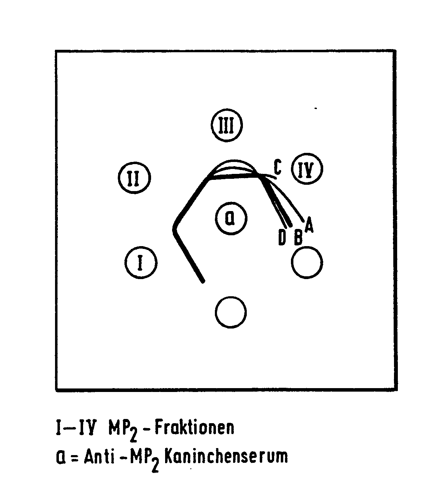

Zum immunchemischen Nachweis der MP$_2$-Proteine mit präzipitierenden Antikörpern kann beispielsweise der Geldiffusionstest nach Ouchterlony verwendet werden. Figur 1 zeigt die immunologische Reaktion der MP$_2$-Fraktionen I—IV mit einem durch Immunisieren von Kaninchen mit Fraktion II gewonnenem Antiserum in diesem Test. Bei den höhermolekularen Fraktionen I und II fallen die Präzipitate gegen die verschiedenen Antigenbezirke weitgehend in einer Linie zusammen, während bei den niedermolekularen Fraktionen III und IV die Präzipitatlinien sich zum Teil voneinander trennen. Die Zuordnung der einzelnen Linien zu den unterschiedlichen Antigenbezirken A, B, C und D ist in Figur 1 gezeigt.

Zum Nachweis der MP$_2$-Proteine oder von damit immunologisch teilverwandten Proteinen können auch empfindlichere immunchemische Methoden wie Radioimmunoassays oder Enzymimmunoassays herangezogen werden.

Der Nachweis und die Bestimmung der MP$_2$-Proteine oder damit teilverwandter Antigene hat diagnostische Bedeutung. Die MP$_2$-Proteine oder damit immunchemisch identische oder verwandte Proteine kommen in bestimmten Organen des Menschen in größerer Konzentration vor; so beispielsweise im embryonalen Magen-und Darmtrakt und in der Niere von erwachsenen Menschen. Bei einer Erkrankung dieser Organe können infolge eines vermehrten Zellerfalls die MP$_2$-Antigene in erhöhter Konzentration im Serum oder im Urin erscheinen. Nachweis und Bestimmung dieser Antigene in Körperflüssigkeiten können daher zur Erkennung von Erkrankungen solcher Organe, zur Überwachung des Krankheitsverlaufs und zur Kontrolle der Therapie eingesetzt werden. In den meisten anderen Organen des Menschen kommen die mit MP$_2$ verwandten Proteine nicht oder nur in Spuren vor.

Die MP$_2$-Proteine können also verwendet werden, um Antiseren und Antikörper herzustellen, die dazu dienen können, MP$_2$-Proteine oder ihre Komponenten mit immunchemischen Methoden nachzuweisen und zu bestimmen.

Andererseits können Antikörper gegen MP$_2$-Proteine und ihre Komponenten zur Herstellung von Immunadsorbentien eingesetzt werden, die dazu dienen können, immunologisch identische oder teilverwandte Proteine zu isolieren. Die Erfindung wird am nachstehenden Beispiel erläutert:

Beispiel
1.1 Zerkleinerung und Waschung der Plazenten

Zur Isolierung der membran-assoziierten Proteine MP$_2$ wurden reife menschliche Plazenten, wie sie bei einer Entbindung anfallen, verwendet. Die Plazenten wurden zunächst bei −20°C eingefroren, im gefrorenen Zustand mit einem Schneidmischer (Cutter) zerkleinert und in dieser Form bei −20°C bis zur Verwendung aufbewahrt. Durch Waschung mit physiologischer NaCl-Lösung wurden zunächst alle löslichen Gewebsproteine entfernt. Dazu wurden 500 g des zerkleinerten Plazentengewebes mit 700 ml NaCl-Lösung versetzt, kurz homogenisiert, dann mehrere Stunden bei 4°C gerührt und schließlich zentrifugiert. Der Überstand wurde verworfen, der Rückstand erneut mit 700 ml NaCl-Lösung mehrere Stunden gerührt und wieder zentrifugiert. Dieser Waschvorgang wurde insgesamt 6 mal wiederholt. Auf diese Weise wurde das Plazentagewebe von löslichen Bestandteilen weitgehend befreit.

1.2 Extraktion des Plazentagewebes mit Triton® X-100

Der Geweberückstand wurde dreimal mit je 700 ml einer Lösung von 2 ml Polyethylenglycol-p-isooctylphenylether (Triton X-100) in 100 ml Wasser extrahiert, wobei jeweils 20 Stunden bei 4°C gerührt und dann abzentrifugiert wurde. Die Extrakte sind zunächst gegen Wasser und dann gegen einen 0,1 mol/l Tris-HCl-Puffer (pH 8,0), der 1 mol/l NaCl und 1 g/l Natriumazid enthielt (Pufferlösung II), dialysiert worden. Die Dialysate wurden auf einem Ultrafilter (Fa. Amicon) unter Verwendung von PM-10 Membranen auf jeweils etwa 200 ml eingeengt und dann vereinigt (Fraktion A).

2. Anreicherung der MP$_2$-Proteine durch Immunadsorption
2.1 Herstellung des Immunadsorbens

Durch Immunisieren von Kaninchen mit der solubilisierten Plazentaprotein-Fraktion A wurden polyvalente Antiseren hergestellt. Sie enthielten Antikörper sowohl gegen die MP$_2$-Proteine als auch gegen andere mit Triton® X-100 löslich gemachte Gewebeantigene. 350 ml eines solchen Antiserumpools wurden gegen 0,02 mol/l Phosphatpuffer (pH 7,0) dialysiert und zur Abtrennung der Immunglobuline mit dem gleichen Puffer an DEAE-Zellulose chromatographiert. Die Immunglobulinfraktion im Durchlauf (3,63 g Protein) wurde dann mit 363 g besonders gereinigter Agarose in Kugelform (Sepharose® von Pharmacia, Uppsala, Schweden), die mit 45,3 g Bromcyan aktiviert worden war, umgesetzt und so kovalent an einen Träger gebunden. Das Verfahren wurde beschrieben von Axen et al., Nature 214 (1967) 1302. Mit Hilfe eines auf diese Weise hergestellten Immunadsorbens konnten die MP$_2$-Proteine zusammen mit anderen solubilisierten Antigenen aus der Plazentafraktion A weiter angerichert werden.

2.2 Durchführung der Immunadsorption

Das Immunadsorbens wurde in Pufferlösung II suspendiert, in eine Chromatographiesäule gefüllt (5,0×20 cm) und mit Pufferlösung II nachgespült. Dann wurde ein Drittel der Fraktion A auf die Säule aufgetragen, wobei die MP$_2$-Proteine und andere solubilisierte Antigene immunadsorptiv gebunden wurden. Die Säule wurde dann gründlich mit Puffer II gespült.

Anschließend sind die adsorbierten Proteine mit etwa 600 ml 6 mol/l Harnstoff-Lösung von der Säule eluiert worden. Die MP$_2$-haltigen Eluate wurden gegen Pufferlösung II dialysiert und mit einem Ultrafilter auf etwa 20 ml eingeengt. Ausbeute pro Adsorption etwa 20—40 mg Einweiß.

Das Adsorbens in der Säule wurde unmittelbar nach der Elution der Proteine wieder mit Pufferlösung II gespült und gründlich gewaschen. Es ist dann erneut zur immunadsorptiven Bindung solubilisierter Antigene eingesetzt worden.

3. Auftrennung der MP$_2$-Proteine durch Gelfiltration

Durch Gelfiltration an Acrylamidagarose AcA-34 (LKB, Stockholm) wurden die immunadsorptiv angereicherten MP$_2$-Proteine aufgrund ihrer unterschiedlichen Molekulargewichte in 4 deutlich voneinander abgesetzte Fraktionen (I—IV) aufgetrennt. Fraktion I kam dabei unmittelbar im Anschluß an das "void volume" von der Säule. Die meisten anderen solubilisierten Membranantigene (MP$_1$ und PP$_4$) besaßen Molekulargewichte unter 200 000 und wurden daher bei der Gelfiltration weitgehend von den MP$_2$-Proteinen abgetrennt. Die MP$_2$-Fraktionen I—IV wurden auf einem Ultrafilter (Fa. Amicon) unter Verwendung von PM-10 Membranen eingeengt.

4. Hochreinigung der MP$_2$-Fraktionen durch inverse Immunadsorption

Die bei der Gelfiltration erhaltenen MP$_2$-Fraktionen waren zum Teil noch durch Spuren von MP$_1$ und Immunglobulinen verunreinigt, während Fraktion III insbesondere noch Ferritin enthielt. Diese Beimengungen konnten durch inverse Immunadsorption, d.h. mit Hilfe von trägergebundenen Antikörpern gegen diese Begleitproteine entfernt werden. Anschließend wurden die gereinigten Proteinlösungen gegen Wasser dialysiert und lyophilisiert.

**Patentansprüche**

1. Proteine MP$_2$, gekennzeichnet durch

a) eine elektrophoretische Beweglichkeit im Bereich zwischen der von α$_2$- und der von β$_1$- Globulinen;

b) einen isoelektrischen Punkt im pH-Bereich von 4,4—5,0;

c) einen Sedimentationskoeffizienten $S^0_{20w}$ im Bereich von 7 bis über 20 S, wobei die Hauptfraktionen (IV, III, II, I) mit etwa 7, 9, 14 bzw. 20 S sedimentieren;

d) Molekulargewichte im Bereich von 200 000 bis 1 000 000, wobei die Hauptfraktionen (IV, III, II, I) Molekulargewichte um 210 000, 400 000, 750 000 und 1 000 000 aufweisen;

e) einen Extinktionskoeffizienten $E^{1\%}_{1\,cm}$ (280 nm) von 12,5±0,2;

f) einen Kohlenhydratanteil von 8,0±3,2% (Mannose 1,4±0,4%, Fucose 0,4±0,2%, Galaktose 1,2±0,4%, N-Acetyl-Glukosamin 2,6±1,8%, N-Acetyl-Neuraminsäure 2,4±1,4%) und

g) eine Aminosäurenzusammensetzung, in der Asparaginsäure, Glutaminsäure, Leucin, Valin, Serin und Glycin zu den am häufigsten vertretenen Aminosäuren gehören.

2. Verfahren zur Gewinnung der Proteine MP$_2$, gekennzeichnet durch

a) eine elektrophoretische Beweglichkeit im Bereich zwischen der von α$_2$- und der von β$_1$-Globulinen;

b) einen isoelektrischen Punkt im pH-Bereich von 4,4—5,0;

c) einen Sedimentationskoeffizienten $S^0_{20w}$ im Bereich von 7 bis über 20 S, wobei die Hauptfraktionen (IV, III, II, I) mit etwa 7, 9, 14 bzw. 20 S sedimentieren;

d) Molekulargewichte im Bereich von 200 000 bis 1 000 000, wobei die Hauptfraktionen (IV, III, II, I) Molekulargewichte um 210 000, 400 000, 750 000 und 1 000 000 aufweisen;

e) einen Extinktionskoeffizienten $E^{1\%}_{1\,cm}$ (280 nm) von 12,5±0,2;

f) einen Kohlenhydratanteil von 8,0±3,2% (Mannose 1,4±0,4%, Fucose 0,4±0,2%, Galaktose 1,2±0,4%, N-Acetyl-Glukosamin 2,6±1,8%, N-Acetyl-Neuraminsäure 2,4±1,4%) und

g) eine Aminosäurenzusammensetzung, in der Asparaginsäure, Glutaminsäure, Leucin, Valin, Serin und Glycin zu den am häufigsten vertretenen Aminosäuren gehören,

dadurch gekennzeichnet, daß man mit physiologischer Salzlösung extrahiertes Plazentenmaterial mit einer 0,5 bis 5 g eines nicht-ionischen Detergenzes auf 100 ml enthaltenden Lösung extrahiert, den Überstand gegen Wasser oder Pufferlösung dialysiert, die in der erhaltenen Lösung enthaltenen solubilisierten Gewebeantigen der Plazenta durch eine Immunadsorption weiter anreichert, die in der erhaltenen Lösung angereicherten Proteine mittels einer Gelfiltration auftrennt und von anderen Proteinen, die ein kleineres Molekulargewicht haben, weitgehend abtrennt und gegebenenfalls noch als Verunreinigungen vorliegende Begleitproteine durch inverse Immunadsorption entfernt und die MP$_2$-Proteine gewinnt.

3. Verwendung der Proteine nach Anspruch 1 zur Herstellung von Antikörpern gegen diese Proteine zum Nachweis und zur Bestimmung dieser Proteine sowie zur Diagnose, Überwachung eines Krankheitsverlaufs oder Therapiekontrolle.

**Revendications**

1. Protéines MP$_2$, caractérisées par

a) une mobilité électrophorétique dans l'intervalle entre celle des $\alpha_2$-globulines et celle des $\beta_1$-globulines;

b) un point isoélectrique dans l'intervalle de pH de 4,4 à 5,0;

c) un coefficient de sédimentation S$^0_{20w}$ dans la plage de 7 à plus de 20 S, les fractions principales (IV, III, II et I) se sédimentant avec des coefficients d'environ 7, 9, 14 ou 20 S;

d) des masses moléculaires dans la plage de 200 000 à 1 000 000, les fractions principales (IV, III, II et I) présentant des masses moléculaires d'environ 210 000, 400 000, 750 000 et 1 000 000;

e) un coefficient d'extinction E$^{1\%}_{1\ cm}$ (280 nm) de 12,5±0,2;

f) une teneur en glucides de 8,0±3,2% (mannose 1,4±0,4%, fucose 0,4±0,2%, galactose 1,2±0,4%, N-acétyl-glucosamine 2,6±1,8%, acide N-acétyl-neuraminique 2,4±1,4%) et

g) une composition en aminoacides dans laquelle l'acide aspartique, l'acide glutamique, la leucine, la valine, la sérine et la glycine appartiennent aux aminoacides les plus fréquemment représentés.

2. Procédé pour l'obtention des protéines MP$_2$, caractérisées par

a) une mobilité électrophorétique dans l'intervalle entre celle des $\alpha_2$-globulines et celle des $\beta_1$-globulines;

b) un point isoélectrique dans l'intervalle de pH de 4,4 à 5,0;

c) un coefficient de sédimentation S$^0_{20w}$ dans la plage de 7 à plus de 20 S, les fractions principales (IV, III, II et I) se sédimentant avec des coefficients d'environ 7, 9, 14 ou 20 S;

d) des masses moléculaires dans la plage de 200 000 à 1 000 000, les fractions principales (IV—I) présentant des masses moléculaires d'environ 210 000, 400 000, 750 000 et 1 000 000;

e) un coefficient d'extinction E$^{1\%}_{1\ cm}$ (280 nm) de 12,5±0,2;

f) une teneur en glucides de 8,0±3,2% (mannose 1,4±0,4%, fucose 0,4±0,2%, galactose 1,2±0,4%, N-acétyl-glucosamine 2,6±1,8%, acide N-acétylneuraminique 2,4±1,4%) et

g) une composition en aminoacides dans laquelle l'acide aspartique, l'acide glutamique, la leucine, la valine, la sérine et la glycine appartiennent aux aminoacides les plus fréquemment représentés,

caractérisé en ce que l'on extrait avec une solution contenant de 0,5 à 5 g d'un détergent non ionique, pour 100 ml, une substance placentaire extraite avec du sérum physiologique, on dialyse le surnageant contre de l'eau ou une solution tampon, on concentre davantage par une immunoadsorption les antigènes tissulaires du placenta, solubilisés, contenus dans la solution obtenue, on scinde au moyen d'une filtration sur gel les protéines concentrées dans la solution obtenue et on les sépare dans une large mesure d'autres protéines qui ont une masse moléculaire plus faible, et éventuellement on élimine par immunoadsorption inversée des protéines accompagnatrices encore présentes en tant qu'impuretés, et on obtient les protéines MP$_2$.

3. Utilisation des protéines selon la revendication 1, pour la production d'anticorps dirigés contre ces protéines, pour la mise en évidence et le dosage de ces protéines ainsi que pour le diagnostic, la surveillance de l'évolution d'une maladie ou le contrôle d'un traitement.

**Claims**

1. Proteins MP$_2$ having the following characteristics:

a) an electrophoretic mobility in the range between that of $\alpha_2$- and that of $\beta_1$- globulins;

b) an isoelectric point in the pH range 4.4—5.0;

c) a sedimentation coefficient S$^0_{20w}$ in the range from 7 to above 20 S, the main fractions (IV, III, II and I) sedimenting at about 7, 9, 14, and 20 S respectively;

d) molecular weights in the range from 200,000 to 1,000,000 the main fractions (IV, III, II, and I) having molecular weights of about 210,000, 400,000, 750,000, and 1,000,000;

e) an extinction coefficient E$^{1\%}_{1\ cm}$ (280 nm) of 12.5±0.2;

f) a carbohydrate content of 8.0±3.2% (mannose 1.4±0.4%, fucose 0.4±0.2%, galactose 1.2±0.4%, N-acetylglucosamine 2.6±1.8%, and N-acetylneuraminic acid 2.4±1.4%) and

g) an amino acid composition in which aspartic acid, glutamic acid, leucine, valine, serine and glycine are among the amino acids present in greatest amounts.

2. A process for obtaining the proteins MP$_2$ which have the following characteristics:

a) an electrophoretic mobility in the range between that of $\alpha_2$—and that of $\beta_1$—globulins;

b) an isoelectric point in the pH range 4.4—5.0;

c) a sedimentation coefficient S$^0_{20w}$ in the range from 7 to above 20 S, the main fractions (IV, III, II and I) sedimenting at about 7, 9, 14, and 20 S respectively;

d) molecular weights in the range from 200,000 to 1,000,000, the main fractions (IV—I) having molecular weights of about 210,000, 400,000, 750,000, and 1,000,000;

e) an extinction coefficient E$^{1\%}_{1\ cm}$ (280 nm) of 12.5±0.2;

f) a carbohydrate content of 8.0±3.2% (mannose 1.4±0.4%, fucose 0.4±0.2%, galactose 1.2±0.4%, N-acetylglucosamine 2.6±1.8%, and N-acetylnewaminic acid 2.4±1.4%) and

g) an aminoacid composition in which aspartic acid, glutamic acid, leucine, valine, serine and glycine are among the amino acids present in greatest amounts,

which comprises extracting placental material, which has been extracted with physiological saline, using a solution containing 0.5 to 5 g of a non-ionic detergent per 100 ml, dialyzing the supernatant against water or buffer solution, further concentrating the solubilized placental tissue antigen, which is contained in the resulting solution, by immunoadsorption, fractionating the concentrated proteins in the resulting solution by gel filtration and substantially removing other proteins which have a smaller molecular weight and, where appropriate, removing accompanying proteins, which are still present as impurities, by inverse immunoadsorption and obtaining the $MP_2$ proteins.

3. The use of the proteins as claimed in claim 1 for the preparation of antibodies against these proteins for the detection and determination of these proteins and for diagnosis, monitoring the progress of a disease or checking therapy.

I—IV MP$_2$ - Fraktionen
a = Anti - MP$_2$ Kaninchenserum